# EUROPEAN PATENT SPECIFICATION

(11) **EP 4 255 414 B1**
(45) Date of publication and mention of the grant of the patent: **05.02.2025**
(21) Application number: 21819506.3
(22) Date of filing: 02.12.2021
(51) Int. Cl.: A61K 31/341, A61P 1/04, A61P 17/00, A61K 9/00, A61K 9/08, A61K 9/14, A61K 9/20

(54) **COMPOSITION CONTAINING RANITIDINE HAVING A LOW IRON CONTENT**
ZUSAMMENSETZUNG MIT RANITIDIN MIT NIEDRIGEM EISENGEHALT
COMPOSITION CONTENANT DE LA RANITIDINE AYANT UNE FAIBLE TENEUR EN FER

(30) Priority: 04.12.2020 EP 20306504
(43) Date of publication of application: 11.10.2023
(73) Proprietor: Opella Healthcare Group SAS, 92200 NEUILLY SUR SEINE (FR)
(72) Inventor: DLUBALA, Alain, 94250 GENTILLY (FR); THOLÉ, France Emmanuelle, 94250 GENTILLY (FR)
(74) Representative: Atout PI Laplace
(86) International application number: PCT/EP2021/084070
(87) International publication number: WO 2022/117771

(56) References cited:
- CN-A- 111 686 087

## Description

### FIELD OF THE INVENTION

The present invention concerns compositions containing ranitidine or one of its pharmaceutically acceptable salts having low iron species content. The present invention also concerns compositions having low content of iron species that are not under complexed form with a chelating agent and/or having low content of iron species that are partially or completely under complexed form with a chelating agent and/or having low content of iron species that are under complexed form with a chelating agent other than ranitidine and/or having low content of complexes formed between ranitidine and iron species. The present invention also concerns a process for the preparation of said compositions. The present invention also concerns the second medical use of said compositions.

### BACKGROUND

Recently, most drug regulatory administrations, in particular the Food and Drug Administration, worldwide have decided to withdraw ranitidine compositions from the market because of concerns over levels of the contaminant N-nitrosodimethylamine (NDMA), which can increase with time and temperature. NDMA has been classified as a probable human carcinogen based on animal studies.

Among many root causes of NDMA contamination in commercial ranitidine compositions, the present inventors have surprisingly found that the NDMA contamination was due to the presence of iron species in said ranitidine compositions.

Without to be bound by a theory, the present inventors consider that the formation of NDMA in ranitidine compositions in the presence of iron species results from the mechanism illustrated by the following scheme.

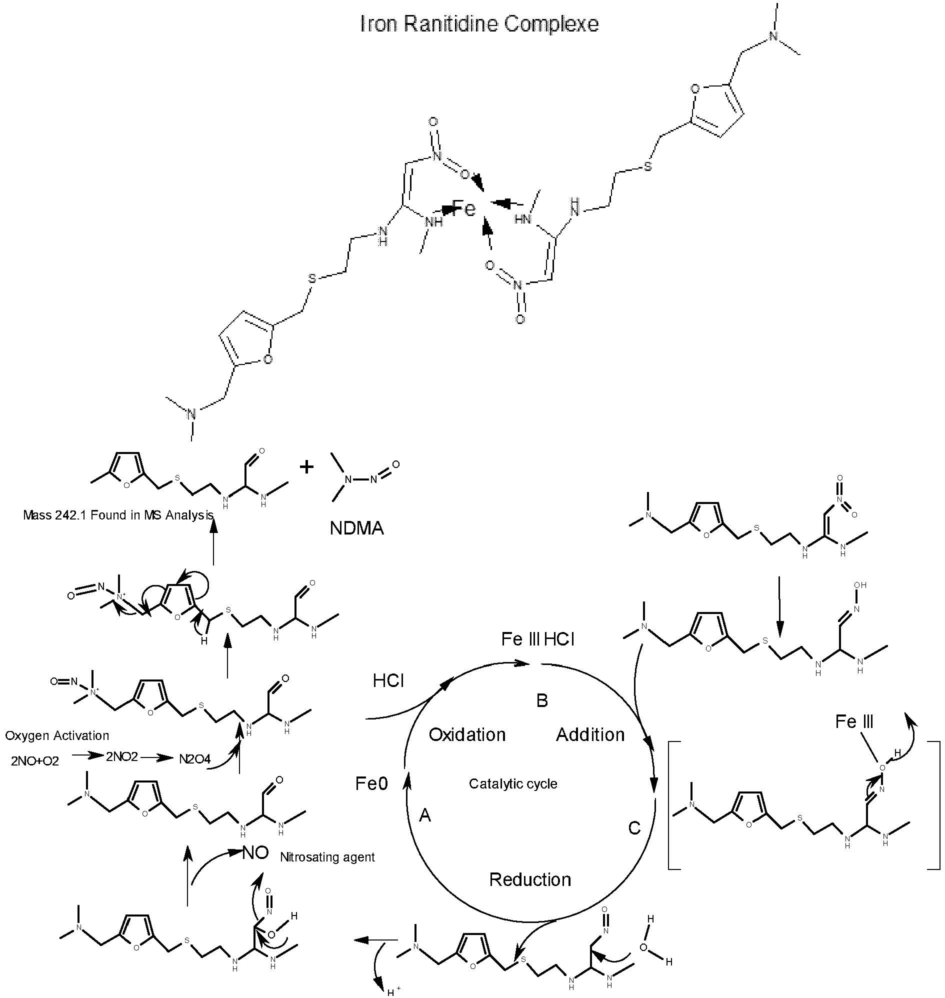

Iron species contamination may come from processing, for example from the apparatus used to produce ranitidine and ranitidine compositions, transportation, or even naturally occurs in the raw material used to produce ranitidine and ranitidine compositions.

The present inventors have also surprisingly found alternatives to reduce the iron species content in compositions containing ranitidine and thus the NMDA content of such compositions even after storage according to stability tests defined in European guidelines CPMP/ICH/2736/99.

CN111686087 discloses a formulation of ranitidine hydrochloride tablets with low NDMA impurities and a process to produce such formulation which does not include contact with iron. The NDMA content of the raw material drug ranitidine hydrochloride used was 0.09 ppm. After processing according to example 1, the NDMA content in the ranitidine hydrochloride tablets was 0.08 ppm, i.e. 80 ppb.

### SUMMARY OF THE INVENTION

A first object of the present invention relates to a composition containing ranitidine or one of its pharmaceutically acceptable salts comprising less than 1 ppm, preferably less than 900 ppb, more preferably less than 600 ppb and even more preferably less than 300 ppb of iron species by weight with respect to the total dry weight of the composition.

A second object of the present invention relates to a composition containing ranitidine or one of its pharmaceutically acceptable salts comprising:
- less than 1 ppm, preferably less than 900 ppb, more preferably less than 600 ppb and even more preferably less than 300 ppb of iron species that are not under complexed form with a chelating agent by weight with respect to the total dry weight of the composition, and/or
- less than 1 ppm, preferably less than 900 ppb, more preferably less than 600 ppb and even more preferably less than 300 ppb of iron species that are partially or completely under complexed form with a chelating agent by weight with respect to the total dry weight of the composition, and/or
- less than 1 ppm, preferably less than 900 ppb, more preferably less than 600 ppb and even more preferably less than 300 ppb of iron species that are under complexed form with a chelating agent other than ranitidine by weight with respect to the total dry weight of the composition, and/or
- less than 1 ppm, preferably less than 900 ppb, more preferably less than 600 ppb and even more preferably less than 300 ppb of complexes formed between ranitidine and iron species by weight with respect to the total dry weight of the composition.

Another object of the present invention relates to a process for the preparation of the compositions as previously defined, wherein said compositions are not brought into contact with iron species.

The compositions as previously defined may be manufactured, at least partly, within a stainless steel free apparatus, a glass apparatus, a glass-line apparatus, an apparatus coated with inactive polymers or copolymers such as copolymers of ethylene and chlorotrifluoroethylene, and polytetrafluoroethylene, and/or an apparatus coated with enamel.

The present invention also relates to a process for the preparation of the composition as previously defined which comprises at least one step selected from a step of complexing at least one part or all the iron species with a chelating agent, a step of removing at least one part or all the complexes formed between a chelating agent and iron species, and a step of removing at least one part or all iron species

Another object of the present invention relates to a composition as previously defined for its use in the prevention and/or treatment of duodenal ulcers, stomach or gastric ulcers, heartburn, indigestion due to acid reflux from the stomach such as gastroesophageal reflux, dyspepsia, dermatitis and Zollinger-Ellison syndrom.

### DETAILED DESCRIPTION

Ranitidine is (*E*)-1-N'-[2-[[5-[(dimethylamino)methyl]furan-2-yl]methylsulfanyl]ethyl]-1-N-methyl-2-nitroethene-1,1-diamine.

By "Ranitidine, HCl" it is herein understood the hydrochloride salts of ranitidine.

By "ppb" it is herein understood part per billion by weight.

By "ppm" it is herein understood part per million by weight.

By "RH" it is herein understood Relative Humidity.

By "Kₛₚ" it is herein understood solubility product constant.

By "purification step" it is herein understood any physical separation of said iron species or complex formed between a chelating agent and iron species from the composition containing ranitidine or its salts. Examples of purification step comprise, but are not limited to, filtration, centrifugation, evaporation, liquid-liquid extraction, crystallization, trituration, absorption, chromatography, smelting, distillation, sublimation, electrolysis, solubilisation, precipitation, magnetic separation, and combination thereof.

By "NDMA" it is herein understood nitrosodimethylamine.

By "at least once" it is herein understood, one time or more, preferably one, two, three, four, five, six, seven, eight, nine, ten, eleven, twelve, thirteen, fourteen, fifteen, sixteen, seventeen, eighteen, nineteen or twenty times, more preferably one, two, three, four, five, six, seven, eight, nine or ten times, and even more preferably one, two, three, four or five times.

The present invention relates to a composition containing ranitidine or one of its pharmaceutically acceptable salts comprising less than 1 ppm, preferably less than 900 ppb, more preferably less than 600 ppb and more preferably less than 300 ppb of iron species by weight with respect to the total dry weight of the composition. For example, the iron species content may be comprised between 900 ppb and 50 ppb, preferably between 600 ppb and 100 ppb and more preferably between 300 ppb and 200 ppb by weight with respect to the total dry weight of the composition.

The present invention also relates to ranitidine or one of its pharmaceutically acceptable salts comprising less than 1 ppm, preferably less than 900 ppb, more preferably less than 600 ppb and more preferably less than 300 ppb of iron species by weight with respect to the total dry weight of the ranitidine or of its pharmaceutically acceptable salts. For example, the iron species content may be comprised between 900 ppb and 50 ppb, preferably between 600 ppb and 100 ppb and more preferably between 300 ppb and 200 ppb by weight with respect to the total dry weight of the ranitidine or of its pharmaceutically acceptable salts.

The iron species content can be measured using techniques well known for the skilled person. Examples of such techniques include, but are not limited to, inductively coupled plasma mass spectrometry (ICP-MS).

In a particular embodiment, said composition comprises less than 320 ppb, preferably less than 160 ppb, more preferably less than 80 ppb and even more preferably less than 17 ppb of N-nitrosodimethylamine (NDMA) by weight with respect to the total dry weight of ranitidine or its pharmaceutically acceptable salts.

In a particular embodiment, said composition comprises less than 320 ppb, preferably less than 160 ppb, more preferably less than 80 ppb and even more preferably less than 17 ppb of N-nitrosodimethylamine (NDMA) by weight with respect to the total dry weight of ranitidine or its pharmaceutically acceptable salts, the content of NDMA being determined according to stability tests defined in European Guidelines CPMP/ICH/2736/99. Preferably, the stability test is the accelerated stability test defined in CPMP/ICH/2736/99 that is 6 months storage at 40°C ± 2°C/75% RH ± 5% RH (Relative Humidity).

In another particular embodiment, said composition comprises less than 320 ppb, preferably less than 160 ppb, more preferably less than 80 ppb and even more preferably less than 17 ppb of N-nitrosodimethylamine (NDMA) by weight with respect to the total dry weight of ranitidine or its pharmaceutically acceptable salts, the content of NDMA being determined after an exposure to 70°C during 5 days. The content of NDMA as previously defined may also be determined after an exposure to forced conditions (that is a temperature superior or equal to about 70°C during 22 days). These exposures can be performed with 75% RH for example.

The NDMA content can be measured using techniques well known for the skilled person. Examples of such techniques include, but are not limited to, gas chromatography mass spectrometry (GC-MS), GC-MS/MS, liquid chromatography mass spectrometry (LC-MS and in particular HPLC-MS) and LC-MS/MS (and in particular HPLC-MS/MS), preferably liquid chromatography mass spectrometry (LC-MS) and LC-MS/MS.

The present invention also relates to a composition containing ranitidine or one of its pharmaceutically acceptable salts comprising:
- less than 1 ppm, preferably less than 900 ppb, more preferably less than 600 ppb and even more preferably less than 300 ppb of iron species that are not under complexed form with a chelating agent by weight with respect to the total dry weight of the composition,
- less than 1 ppm, preferably less than 900 ppb, more preferably less than 600 ppb and even more preferably less than 300 ppb of iron species that are partially or completely under complexed form with a chelating agent by weight with respect to the total dry weight of the composition, and/or
- less than 1 ppm, preferably less than 900 ppb, more preferably less than 600 ppb and even more preferably less than 300 ppb of iron species that are under complexed form with a chelating agent other than ranitidine by weight with respect to the total dry weight of the composition, and/or
- less than 1 ppm, preferably less than 900 ppb, more preferably less than 600 ppb and even more preferably less than 300 ppb of complexes formed between ranitidine and iron species by weight with respect to the total dry weight of the composition.

For example, the content of iron species that are not under complexed form with a chelating agent may be comprised between 900 ppb and 50 ppb, preferably between 600 ppb and 100 ppb and more preferably between 300 ppb and 200 ppb by weight with respect to the total dry weight of the composition.

For example, the content of iron species that are partially or completely under complexed form with a chelating agent may be comprised between 900 ppb and 50 ppb, preferably between 600 ppb and 100 ppb and more preferably between 300 ppb and 200 ppb by weight with respect to the total dry weight of the composition.

For example, the content of iron species that are under complexed form with a chelating agent other than ranitidine may be comprised between 900 ppb and 50 ppb, preferably between 600 ppb and 100 ppb and more preferably between 300 ppb and 200 ppb by weight with respect to the total dry weight of the composition.

For example, the content of complexes formed between ranitidine and iron species may be comprised between 900 ppb and 50 ppb, preferably between 600 ppb and 100 ppb and more preferably between 300 ppb and 200 ppb by weight with respect to the total dry weight of the composition.

The content of iron species that are not under complexed form with a chelating agent can be measured using techniques well known for the skilled person. Examples of such techniques include, but are not limited to, inductively coupled plasma mass spectrometry (ICP-MS).

The content of iron species that are partially or completely under complexed form can be measured using techniques well known for the skilled person. Examples of such techniques include, but are not limited to, inductively coupled plasma mass spectrometry (ICP-MS).

The content of iron species that are under complexed form with a chelating agent other than ranitidine can be measured using techniques well known for the skilled person. Examples of such techniques include, but are not limited to, inductively coupled plasma mass spectrometry (ICP-MS).

The content of complexes formed between ranitidine and iron species can be measured using techniques well known for the skilled person. Examples of such techniques include, but are not limited to, inductively coupled plasma mass spectrometry (ICP-MS).

The pharmaceutically acceptable salt of ranitidine as previously defined may be ranitidine hydrochloride, ranitidine bismuth citrate, and mixtures thereof.

The iron specie as previously defined may be metallic iron (iron (0)), iron (II) and iron (III) and mixtures thereof, preferably metallic iron (iron (0)), iron oxide such as iron (II) oxide and iron (III) oxide, iron chloride such as iron (II) chloride and iron (III) chloride, and mixtures thereof.

Preferably, the compositions as previously defined comply with pharmacopoeia, in particular with the United States Pharmacopeia or the European Pharmacopoeia.

The compositions as previously defined may also comprise one or more excipients.

The compositions as previously defined may consist of ranitidine or its pharmaceutically acceptable salts with iron species in the amounts as previously defined.

The compositions as previously defined may essentially consist of ranitidine or its pharmaceutically acceptable salts with iron species in the amounts as previously defined.

The compositions as previously defined may also consist of ranitidine or its pharmaceutically acceptable salts with iron species in the amounts as previously defined and one or more excipients.

The compositions as previously defined may also essentially consist of ranitidine or its pharmaceutically acceptable salts with iron species in the amounts as previously defined and one or more excipients.

The ranitidine may be in the form of a powder, a compacted powder or a solution.

The compositions as previously defined may be pharmaceutical compositions.

The present invention also relates to a process for the preparation of the compositions as previously defined.

In a particular embodiment, during the preparation process said compositions are not brought into contact with iron species.

The compositions as previously defined may be manufactured, at least partly, within a stainless steel free apparatus, a glass apparatus, a glass-line apparatus, an apparatus coated with inactive polymers or copolymers such as copolymers of ethylene and chlorotrifluoroethylene, and polytetrafluoroethylene, and/or an apparatus coated with enamel.

The term "inactive polymers" refers to inert polymers that is polymers that do not leave metallic residues such as iron species and are resistant to hot corrosive environments such as hydrochloric acid and ethanol at high temperatures. Thus, inactive polymers enable to ensure the permanence and continuity of the protective polymer film bonded to the metal (as stainless steel) of the apparatus. Examples of inactive polymers comprise, but are not limited to, copolymers of ethylene and chlorotrifluoroethylene, polytetrafluoroethylene, and mixtures thereof.

Examples of copolymers of ethylene and chlorotrifluoroethylene comprise, but are not limited to, Halar^{®} commercialized by Solvay.

The process for the preparation of the compositions as previously defined may comprise at least one step selected from a step of complexing at least one part or all the iron species with a chelating agent, a step of removing at least one part or all the complexes formed between a chelating agent and iron species, and a step of removing at least one part or all iron species.

In particular, the at least one step selected from a step of complexing at least one part or all the iron species with a chelating agent, a step of removing at least one part or all the complexes formed between a chelating agent and iron species, and a step of removing at least one part or all iron species comprises:
- a distilling step of methylthiomethyl (MTM) compounds such as 1-methylamino-1-methylthio-2-nitroethene, and/or
- a complexation step by using N,N-dialkyl-dithiocarbamate such as N,N-dimethyldithiocarbamate, N,N-diethyl-dithiocarbamate and/or N,N-dibutyl-dithiocarbamate, ethylenediaminetetraacetic acid (EDTA) and/or cysteamine, optionally followed by a purification step, and/or
- a complexation step by using a resin or silica grafted with N,N-dialkyl-dithiocarbamate such as N,N-dimethyl-dithiocarbamate, N,N-diethyl-dithiocarbamate and/or N,N-dibutyldithiocarbamate, ethylenediaminetetraacetic acid (EDTA) and/or cysteamine, optionally followed by a purification step, and/or
- a complexation step by using a resin or silica grafted with a carbon chain terminated by a functional group which is an iron chelating agent optionally followed by a purification step, and/or
- an adsorption step by using zeolite, activated carbon, silica or silica activated carbon composite, and/or
- a separation step by using magnetic separators.

These steps can be combined and/or repeated if necessary.

The iron chelating agent functional group as previously defined may be selected from a dithiocarbamate group, a thiol group, a dimethoxytrityl (DMT) group, an amine group, an (aminomethyl)phosphonic acid (AMPA) group, a cysteine group, a propyldiethanolamine (DEAM) group, a diamine group, a dodecane-tetraacetic acid (DOTA) group, an imidazole group, a triaminetetraacetic acid (TAAcOH) group, a sodium triaminetetraacetate (TAACONa) group, a thiourea group, a p-toluenesulfonic acid (tosic acid) group, a triamine group, and mixtures thereof.

The silica grafted with a carbon chain terminated by a functional group may be iron scavengers commercialised by SiliCycle under the tradename SiliaMetS^{®} such as SiliaMetS^{®} Cysteine (Si-Cys) which is the silica bound equivalent of the amino acid Cysteine.

The complex formed between iron species and the chelating agent can be present in the final composition or can be removed from the composition by a purification step such as a filtration step. In case chelation of iron species inactivates iron species, that is iron species once complexed can no longer be involed in oxidation-reduction reactions, the complex formed between iron species and the chelating agent can be present in the final composition. Preferably, the complex formed between iron species and the chelating agent is removed from the composition by a purification step such as a filtration step.

It is known that methylthiomethyl (MTM) compounds such as 1-methylamino-1-methylthio-2-nitroethene have a high thermal stability (no decomposition before 190°C). Thus, by using methylthiomethyl (MTM) compounds such as 1-methylamino-1-methylthio-2-nitroethene in a distillation process, the iron species can be separated from ranitidine. The distillation step can be carried out under reduced pressure, in particular in the range of millibar.

The complex formed between chelating agents such as N,N-dialkyl-dithiocarbamate, EDTA or cysteamine and iron species can be easily separated from a solid composition containing ranitidine by a purification step such as a filtration step due to solubility differences between said complex and ranitidine.

For example, the complex formed between N,N-dialkyl-dithiocarbamate and iron species has a low solubility in water and thus a high pKₛₚ (equal to - log(Kₛₚ)) contrary to ranitidine. Thus, the complex formed between N,N-dialkyl-dithiocarbamate and iron species can be easily separated from the composition containing ranitidine by a purification step such as a filtration step.

In the same manner, due to solubility properties of the complex formed between EDTA and iron species, said complex can be easily separated from the composition containing ranitidine by a purification step such as a filtration step.

Cysteamine is an iron chelating agent. The present inventors have found that cysteamine which is a reagent of the synthesis of ranitidine can be an iron chelating agent if it is present in excess. The cysteamine can thus be added in the manufacturing process of ranitidine or a crude adduct of cysteamine and methylthiomethyl (MTM) (in particular 2-(((5-((dimethylamino)methyl)furan-2-yl)methyl)thio)ethylamine also named cystofur or cystofer) can be used. The use of cysteamine as a chelating agent enables avoiding the use of additional chemical entities within the process and is thus advantageous. Thus, preferably the iron chelating agent is cysteamine.

Examples of adsorption step of iron species by using activated carbon, silica or silica activated carbon composite are described in Heavy Metals Removal Using Activated Carbon, Silica and Silica Activated Carbon Composite, Mona Karnib et al., Energy Procedia 50 (2014) 113 - 120*.* Zeolithes, activated carbon, silica or silica activated carbon composite can be used during purification such as filtration of a homogeneous phase containing ranitidine in a solvent such as ethyl acetate.

Due to ferromagnetic properties of the complexes formed between ranitidine and some or all iron species such as iron (0) and iron (III) and of some or all iron species such as iron (0) and iron (III), such compounds can be captured by using magnetic field, in particular by using magnetic separators. Magnetic separation equipment that traps iron species can comprise ceramic, neodymium iron boron (neodymium), praseodym and/or neodym.

A process of removing at least one part or all iron species may also comprise the following steps:
a) a dissolution step of ranitidine in a solvent such as water followed by a purification step such as a purification step by reverse osmosis or followed by a filtration step such as ultrafiltration,
b) optionally a recrystallization step using a solvent such as an alcohol based solvent and preferably methanol, ethanol and/or isopropanol.

Step a) may be repeated at least once before step b). Steps a) and b) may be repeated at least once.

The processes as previously defined can be used in combination.

High temperature, high humidity and oxygen influence the degradation rate of ranitidine. Also, low pH influences the degradation rate of ranitidine. Furthermore, chemical activity of ranitidine in a heterogeneous solid phase is lower than in a homogeneous aqueous phase.

Thus, the compositions as previously defined may be manufactured and/or stored under atmosphere having a low oxygen content, preferably said compositions are manufactured and/or stored are under inert atmosphere by using inert gas such as nitrogen, argon and mixtures thereof.

The compositions as previously defined may also be manufactured and/or stored at low temperatures.

The compositions as previously defined may also be manufactured and/or stored under low relative humidity.

The compositions as previously defined may also be manufactured and/or stored with low water and ethanol contents.

The compositions as previously defined may also be manufactured and/or stored at low pH.

The compositions as previously defined may also be stored under a solid form.

It is known that ranitidine can be used in the prevention and/or treatment of duodenal ulcers, stomach or gastric ulcers, heartburn, indigestion due to acid reflux from the stomach such as gastroesophageal reflux, dyspepsia, dermatitis and Zollinger-Ellison syndrom.

Thus, another object of the present invention relates to the compositions as previously defined for their use in the prevention and/or treatment of duodenal ulcers, stomach or gastric ulcers, heartburn, indigestion due to acid reflux from the stomach such as gastroesophageal reflux, dyspepsia, dermatitis and Zollinger-Ellison syndrom.

### DESCRIPTION OF FIGURE

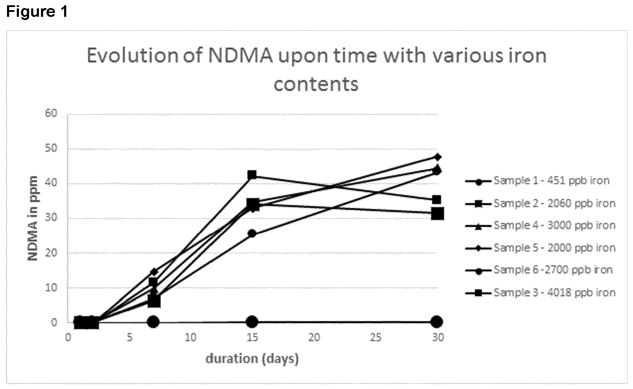

Figure 1 represents the NDMA content in ranitidine samples after 1, 2, 7, 15 and 30 days of storage at 40°C.

### EXAMPLES

### Example 1: Iron spiking

This experiment aims to demonstrate that the presence of iron species in commercially available compositions containing ranitidine (not according to the invention) leads to the degradation of ranitidine to NDMA.

### Protocol:

The tests are described in table 1 below.

**Table 1**

| | **Sample** | **T (°C)** | **Time** | **Atmosphere** | **Spiking** | **Solvent** | **NDMA (ppm)** |
|---|---|---|---|---|---|---|---|
| Spiking with Fe mixture in a solid state | Commercial Ranitidine HCl (not according to the present invention) | 70 | 24h | Air | 1% molar Fe(O) | - | **107** |
| | | | | | 1% molar Fe(II) | | |
| | | | | | 1% molar Fe(III) | | |
| | Commercial Ranitidine HCl (not according to the present invention) | 70 | 24h | Nitrogen | 1% molar Fe(O) | - | **11** |
| | | | | | 1% molar Fe(II) | | |
| | | | | | 1% molar Fe(III) | | |
| No spiking in a solid state | Commercial Ranitidine HCl (not according to the present invention) | 70 | 5 days | Air | - | - | **< 10** |
| | Commercial Ranitidine HCl (not according to the present invention) | 70 | 5 days | Nitrogen | - | - | **< 10** |

The ranitidine HCl used in these tests is a current representative industrial sample of ranitidine HCl having an iron species content of around 1500 ppb (1,5 ppm) (not according to the present invention).

To ensure complete homogenisation, the mixtures have been grinded in a mortar.

The NDMA content has been measured using GC-MC measurement method.

### Results:

The results are presented in table 1.

As shown in table 1:
- The presence of iron species promotes the formation of NDMA in the tested ranitidine, HCl compositions.
- The exposure to oxygen of the ranitidine, HCl tested compositions promotes the formation of NDMA since strong interactions as iron/oxygen further activate the NDMA formation process. By contrast, in nitrogen atmosphere even with iron spiking the NDMA formation process is strongly slowed down but not cancelled. Oxygen is thus a very important factor for the formation of NDMA in the presence of iron in ranitidine compositions. Indeed, the nitrosating N₂O species (dimerised in N₂O4) is present with oxygen and not with nitrogen where only the precursor NO, which is known to be less active in nitrosation reaction, is present.

Iron (Iron 0, II, III) has a greater chemical activity in a heterogeneous solid phase than in a homogeneous aqueous phase (not shown). This is due to a higher pH in the solid form than in the form dissolved in water.

The presence of a mixture of iron species is more active than the stainless-steel pieces (not shown). Indeed, there is a synergetic effect between the species known in the cleavage of ethene-nitro into aldehyde and nitroxide with the Nef reaction where iron (0) contains a small quantity of iron (III) to start the reduction cycle and therefore the cleavage cycle.

A low-temperature test would have also demonstrated the importance of the temperature factor. Indeed, it has been previously observed that at 70°C NDMA formation was more important (in ppm range) whereas at 40°C it was less important (ppb range) (not shown) which justifies the use of the temperature of 70°C to allow the quantification of NDMA by using GC-MS system compatible with quantification in the ppb range.

### Conclusion:

The degradation of Ranitidine, HCl to NDMA in the presence of iron species and oxygen is confirmed. The phenomenon can be amplified in an aqueous phase due to a low pH promoting the degradation reaction (not shown).

Thus, obtaining a Ranitidine, HCl with low content of iron species as defined in the present invention make it possible to guarantee a Ranitidine, HCl with a low content of NDMA after being stored in the conditions defined in CPMP/ICH/2736/99 (stability test 6 months storage at 40°C, 75% RH).

### Example 2: Mechanism of formation of NDMA

As previously indicated, the present inventors have surprisingly found that the formation of NDMA in ranitidine compositions was due to the presence of iron species in said ranitidine compositions and that the mechanism of NDMA formation could be illustrated by the following scheme.

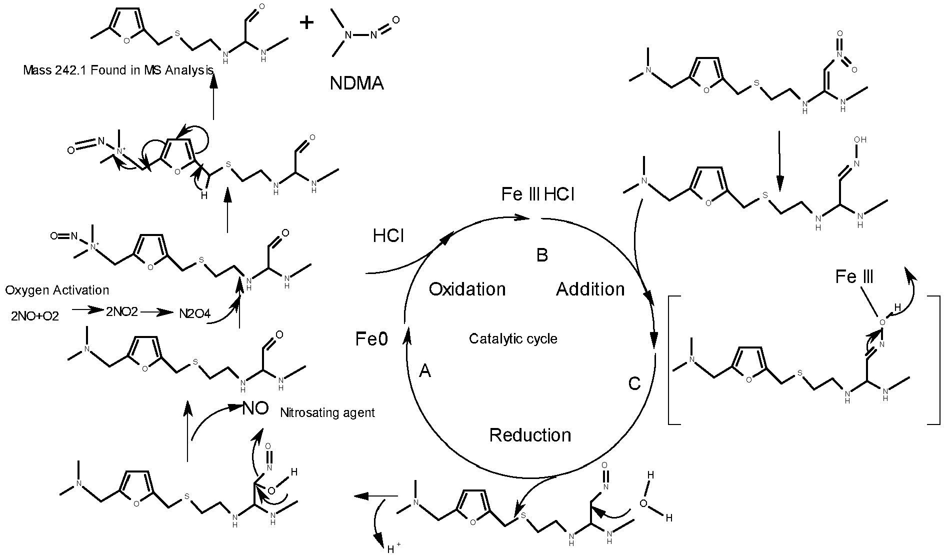

HPLC-MS and HPLC-MS/MS analysis have been performed thereby demonstrating the above-mentioned NDMA formation mechanism in the presence of iron species in ranitidine compositions.

In particular, HPLC-MS/MS analysis results showed that a key intermediate having an enolizable aldehyde moiety, obtained after the cleavage of the nitrite moiety by iron species, was involved in the formation of NDMA (results not shown).

This finding demonstrates that NDMA formation is due to iron species presence and includes the following steps: cleavage of ranitidine with iron species, concomitant formation of mineral nitrite, NDMA formation, the NDMA formation being accelerated when the temperature increases.

### Example 3: Correlation between iron content and NDMA formation

Ranitidine samples containing iron species in amounts indicated in table 2 have been prepared.

NDMA contents in ranitidine samples have been measured by HPLC-MS after 1, 2, 7, 15 and 30 days of storage at 40°C.

Total iron contents in ranitidine samples have been measured by ICP-MS.

Results are presented in table 2 and on figure 1.

**Table 2**

| | | | | **NDMA (ppm) after x day(s) at 40°C** | | | | |
|---|---|---|---|---|---|---|---|---|
| **Sample number** | **T°C** | **Iron (ppb)** | **ethanol (ppm)** | **1** | **2** | **7** | **15** | **30** |
| Sample 1 | 40 | 451 | 279 | 0,01 | 0,01 | 0,03 | 0,062 | 0,153 |
| Sample 2 | 40 | 2060 | | 0,01 | 0,051 | 6,41 | 34,109 | 31,48 |
| Sample 3 | 40 | 4018 | | 0,01 | 0,03 | 11,53 | 42,238 | 35,13 |
| Sample 4 | 40 | 3000 | | 0,03 | 0,038 | 9,87 | 34,664 | 44,53 |
| Sample 5 | 40 | 2000 | | 0,03 | 0,029 | 14,66 | 32,949 | 47,68 |
| Sample 6 | 40 | 2700 | | 0,03 | 0,102 | 7,13 | 25,324 | 43,35 |

These results demonstrate a correlation between iron content and NDMA formation.

## Claims

1. Composition containing ranitidine or one of its pharmaceutically acceptable salts comprising less than 1 ppm, preferably less than 900 ppb, more preferably less than 600 ppb and even more preferably less than 300 ppb of iron species by weight with respect to the total dry weight of the composition.

2. Composition containing ranitidine or one of its pharmaceutically acceptable salts comprising:
- less than 1 ppm, preferably less than 900 ppb, more preferably less than 600 ppb and even more preferably less than 300 ppb of iron species that are not under complexed form with a chelating agent by weight with respect to the total dry weight of the composition, and/or
- less than 1 ppm, preferably less than 900 ppb, more preferably less than 600 ppb and even more preferably less than 300 ppb of iron species that are partially or completely under complexed form with a chelating agent by weight with respect to the total dry weight of the composition, and/or
- less than 1 ppm, preferably less than 900 ppb, more preferably less than 600 ppb and even more preferably less than 300 ppb of iron species that are under complexed form with a chelating agent other than ranitidine by weight with respect to the total dry weight of the composition, and/or
- less than 1 ppm, preferably less than 900 ppb, more preferably less than 600 ppb and even more preferably less than 300 ppb of complexes formed between ranitidine and iron species by weight with respect to the total dry weight of the composition.

3. Composition according to claim 1 or 2, wherein said composition comprises less than 320 ppb, preferably less than 160 ppb, more preferably less than 80 ppb and even more preferably less than 17 ppb of N-nitrosodimethylamine (NDMA) by weight with respect to the total dry weight of ranitidine or its pharmaceutically acceptable salts after an exposure to 70°C during 5 days.

4. Composition according to anyone of claims 1 to 3, wherein the pharmaceutically acceptable salt of ranitidine is ranitidine hydrochloride.

5. Composition according to anyone of claims 1 to 4, wherein the iron specie is metallic iron, iron (II) and iron (III) and mixtures thereof, preferably metallic iron, iron oxide such as iron (II) oxide and iron (III) oxide, iron chloride such as iron (II) chloride and iron (III) chloride, and mixtures thereof.

6. Composition according to anyone of claims 1 to 5, which is a pharmaceutical composition.

7. Process for the preparation of the composition as defined in anyone of claims 1 to 6, wherein said composition is not brought into contact with iron species.

8. Process for the preparation of the composition as defined in anyone of claims 1 to 6, wherein said composition is manufactured, at least partly, within a stainless steel free apparatus, a glass apparatus, a glass-line apparatus, an apparatus coated with inactive polymers or copolymers such as copolymers of ethylene and chlorotrifluoroethylene, and polytetrafluoroethylene, and/or an apparatus coated with enamel.

9. Process for the preparation of the composition as defined in anyone of claims 1 to 6, which comprises at least one step selected from a step of complexing at least one part or all the iron species with a chelating agent, a step of removing at least one part or all the complexes formed between a chelating agent and iron species, and a step of removing at least one part or all iron species.

10. Process according to claim 9, wherein the at least one step selected from a step of complexing at least one part or all the iron species with a chelating agent, a step of removing at least one part or all the complexes formed between a chelating agent and iron species, and a step of removing at least one part or all iron species comprises:
- a distilling step of methylthiomethyl (MTM) compounds such as 1-methylamino-1-methylthio-2-nitroethene, and/or
- a complexation step by using N,N-dialkyl-dithiocarbamate such as N,N-dimethyldithiocarbamate, N,N-diethyl-dithiocarbamate and/or N,N-dibutyl-dithiocarbamate, ethylenediaminetetraacetic acid (EDTA) and/or cysteamine, optionally followed by a purification step, and/or
- a complexation step by using a resin or silica grafted with N,N-dialkyl-dithiocarbamate such as N,N-dimethyl-dithiocarbamate, N,N-diethyl-dithiocarbamate and/or N,N-dibutyl-dithiocarbamate, ethylenediaminetetraacetic acid (EDTA) and/or cysteamine, optionally followed by a purification step, and/or
- a complexation step by using a resin or silica grafted with a carbon chain terminated by a functional group which is an iron chelating agent optionally followed by a purification step, and/or
- an adsorption step by using zeolite, activated carbon, silica or silica activated carbon composite, and/or
- a separation step by using magnetic separators.

11. Process according to claim 10, wherein the iron chelating agent functional group is selected from a dithiocarbamate group, a thiol group, a dimethoxytrityl (DMT) group, an amine group, an (aminomethyl)phosphonic acid (AMPA) group, a cysteine group, a propyldiethanolamine (DEAM) group, a diamine group, a dodecane-tetraacetic acid (DOTA) group, an imidazole group, a triaminetetraacetic acid (TAAcOH) group, a sodium triaminetetraacetate (TAACONa) group, a thiourea group, a p-toluenesulfonic acid, a triamine group, and mixtures thereof.

12. Process according to claim 10, wherein silica grafted with a carbon chain terminated by a functional group are iron scavengers commercialised by SiliCycle under the tradename SiliaMetS^{®}.

13. Composition according to anyone of claims 1 to 6, for its use in the prevention and/or treatment of duodenal ulcers, stomach or gastric ulcers, heartburn, indigestion due to acid reflux from the stomach such as gastroesophageal reflux, dyspepsia, dermatitis and Zollinger-Ellison syndrom.

## Patentansprüche

1. Zusammensetzung, die Ranitidin oder eines seiner pharmazeutisch verträglichen Salze enthält und die weniger als 1 ppm, vorzugsweise weniger als 900 ppb, noch bevorzugter weniger als 600 ppb und noch stärker bevorzugt weniger als 300 ppb nach Gewicht an Eisenspezies, bezogen auf das Gesamttrockengewicht der Zusammensetzung, umfasst.

2. Zusammensetzung, die Ranitidin oder eines seiner pharmazeutisch verträglichen Salze enthält und Folgendes umfasst:
- weniger als 1 ppm, vorzugsweise weniger als 900 ppb, noch bevorzugter weniger als 600 ppb und noch stärker bevorzugt weniger als 300 ppb nach Gewicht an Eisenspezies, die nicht in komplexierter Form mit einem Chelatbildner vorliegen, bezogen auf das Gesamttrockengewicht der Zusammensetzung, und/oder
- weniger als 1 ppm, vorzugsweise weniger als 900 ppb, noch bevorzugter weniger als 600 ppb und noch stärker bevorzugt weniger als 300 ppb nach Gewicht an Eisenspezies, die teilweise oder vollständig in komplexierter Form mit einem Chelatbildner vorliegen, bezogen auf das Gesamttrockengewicht der Zusammensetzung, und/oder
- weniger als 1 ppm, vorzugsweise weniger als 900 ppb, noch bevorzugter weniger als 600 ppb und noch stärker bevorzugt weniger als 300 ppb nach Gewicht an Eisenspezies, die in komplexierter Form mit einem anderen Chelatbildner als Ranitidin vorliegen, bezogen auf das Gesamttrockengewicht der Zusammensetzung, und/oder
- weniger als 1 ppm, vorzugsweise weniger als 900 ppb, noch bevorzugter weniger als 600 ppb und noch stärker bevorzugt weniger als 300 ppb nach Gewicht an zwischen Ranitidin und Eisenspezies gebildeten Komplexen, bezogen auf das Gesamttrockengewicht der Zusammensetzung.

3. Zusammensetzung nach Anspruch 1 oder 2, wobei die Zusammensetzung weniger als 320 ppb, vorzugsweise weniger als 160 ppb, noch bevorzugter weniger als 80 ppb und noch stärker bevorzugt weniger als 17 ppb N-Nitrosodimethylamin (NDMA) nach Gewicht, bezogen auf das Gesamttrockengewicht von Ranitidin oder seinen pharmazeutisch verträglichen Salzen, nach einer Einwirkung von 5 Tagen bei 70 °C umfasst.

4. Zusammensetzung nach einem der Ansprüche 1 bis 3, wobei das pharmazeutisch verträgliche Salz von Ranitidin Ranitidinhydrochlorid ist.

5. Zusammensetzung nach einem der Ansprüche 1 bis 4, wobei die Eisenspezies metallisches Eisen, Eisen (II) und Eisen (III) und Mischungen davon ist, vorzugsweise metallisches Eisen, Eisenoxid wie Eisen(II)-oxid und Eisen(III)-oxid, Eisenchlorid wie Eisen(II)-chlorid und Eisen(III)-chlorid und Mischungen davon.

6. Zusammensetzung nach einem der Ansprüche 1 bis 5, die eine pharmazeutische Zusammensetzung ist.

7. Verfahren zur Herstellung der wie in einem der Ansprüche 1 bis 6 definierten Zusammensetzung, wobei die Zusammensetzung nicht mit Eisenspezies in Kontakt gebracht wird.

8. Verfahren zur Herstellung der wie in einem der Ansprüche 1 bis 6 definierten Zusammensetzung, wobei die Zusammensetzung mindestens teilweise in einem Apparat frei von rostfreiem Stahl, einem Glasapparat, einem Apparat mit Glasleitung, einem Apparat, der mit inaktiven Polymeren oder Copolymeren wie Copolymeren von Ethylen und Chlortrifluorethylen und Polytetrafluorethylen beschichtet ist, und/oder einem mit Emaille beschichteten Apparat hergestellt wird.

9. Verfahren zur Herstellung der wie in einem der Ansprüche 1 bis 6 definierten Zusammensetzung, das mindestens einen Schritt umfasst, der ausgewählt ist aus einem Schritt des Komplexierens mindestens eines Teils der oder aller Eisenspezies mit einem Chelatbildner, einem Schritt des Entfernens mindestens eines Teils der oder aller zwischen einem Chelatbildner und Eisenspezies gebildeten Komplexe und einem Schritt des Entfernens mindestens eines Teils der oder aller Eisenspezies.

10. Verfahren nach Anspruch 9, wobei der mindestens eine Schritt, der aus einem Schritt des Komplexierens mindestens eines Teils der oder aller Eisenspezies mit einem Chelatbildner, einem Schritt des Entfernens mindestens eines Teils der oder aller zwischen einem Chelatbildner und Eisenspezies gebildeten Komplexe und einem Schritt des Entfernens mindestens eines Teils der oder aller Eisenspezies ausgewählt ist, Folgendes umfasst:
- einen Destillationsschritt von Methylthiomethyl- (MTM)-Verbindungen wie 1-Methylamino-1-methylthio-2-nitroethen, und/oder
- einen Komplexierungsschritt unter Verwendung von N,N-Dialkyldithiocarbamat wie N,N-Dimethyldithiocarbamat, N,N-Diethyldithiocarbamat und/oder N,N-Dibutyldithiocarbamat, Ethylendiamintetraessigsäure (EDTA) und/oder Cysteamin, optional gefolgt von einem Reinigungsschritt, und/oder
- einen Komplexierungsschritt unter Verwendung eines Harzes oder Siliciumdioxids, das mit N,N-Dialkyldithiocarbamat wie N,N-Dimethyldithiocarbamat, N,N-Diethyldithiocarbamat und/oder N,N-Dibutyldithiocarbamat, Ethylendiamintetraessigsäure (EDTA) und/oder Cysteamin gepfropft ist, optional gefolgt von einem Reinigungsschritt, und/oder
- einen Komplexierungsschritt unter Verwendung eines Harzes oder Siliciumdioxids, das mit einer Kohlenstoffkette gepfropft ist, an deren Ende eine funktionelle Gruppe steht, die ein Eisenchelatbildner ist, optional gefolgt von einem Reinigungsschritt, und/oder
- einen Adsorptionsschritt unter Verwendung von Zeolith, Aktivkohle, Siliziumdioxid oder einem Siliziumdioxid-Aktivkohle-Verbundstoff, und/oder
- einen Trennungsschritt unter Verwendung von Magnetabscheidern.

11. Verfahren nach Anspruch 10, wobei die funktionelle Gruppe des Eisenchelatbildners ausgewählt ist aus einer Dithiocarbamatgruppe, einer Thiolgruppe, einer Dimethoxytrityl (DMT)-Gruppe, einer Amingruppe, einer (Aminomethyl)phosphonsäure (AMPA)-Gruppe, einer Cysteingruppe, einer Propyldiethanolamin (DEAM)-Gruppe, einer Diamingruppe, einer Dodecantetraessigsäure (DOTA)-Gruppe, einer Imidazolgruppe, einer Triamin-tetraessigsäure (TAAcOH)-Gruppe, einer Natriumtriamintetraacetat (TAACONa)-Gruppe, einer Thioharnstoffgruppe, einer p-Toluolsulfonsäure, einer Triamingruppe und Mischungen davon.

12. Verfahren nach Anspruch 10, wobei es sich bei dem Siliciumdioxid, das mit einer Kohlenstoffkette gepfropft ist, an deren Ende eine funktionelle Gruppe steht, um Eisenfänger handelt, die von SiliCycle unter dem Handelsnamen SiliaMetS^{®} vertrieben werden.

13. Zusammensetzung nach einem der Ansprüche 1 bis 6 zur Verwendung bei der Vorbeugung und/oder Behandlung von Zwölffingerdarmgeschwüren, Magen- oder gastrischen Geschwüren, Sodbrennen, einer Verdauungsstörung aufgrund von Säurerückfluss aus dem Magen wie gastroösophagealem Reflux, Dyspepsie, Dermatitis und Zollinger-Ellison-Syndrom.

## Revendications

1. Composition contenant de la ranitidine ou un de ses sels pharmaceutiquement acceptables et comprenant moins de 1 ppm, de préférence moins de 900 ppb, de manière davantage préférée moins de 600 ppb, et de manière encore davantage préférée moins de 300 ppb d'espèces de fer en poids par rapport au poids sec total de la composition.

2. Composition contenant de la ranitidine ou un de ses sels pharmaceutiquement acceptables et comprenant :
- moins de 1 ppm, de préférence moins de 900 ppb, de manière davantage préférée moins de 600 ppb, et de manière encore davantage préférée moins de 300 ppb d'espèces de fer qui ne sont pas sous forme complexée avec un agent chélatant en poids par rapport au poids sec total de la composition, et/ou
- moins de 1 ppm, de préférence moins de 900 ppb, de manière davantage préférée moins de 600 ppb, et de manière encore davantage préférée moins de 300 ppb d'espèces de fer qui sont partiellement ou totalement sous forme complexée avec un agent chélatant en poids par rapport au poids sec total de la composition, et/ou
- moins de 1 ppm, de préférence moins de 900 ppb, de manière davantage préférée moins de 600 ppb, et de manière encore davantage préférée moins de 300 ppb d'espèces de fer qui sont sous forme complexée avec un agent chélatant autre que la ranitidine en poids par rapport au poids sec total de la composition, et/ou
- moins de 1 ppm, de préférence moins de 900 ppb, de manière davantage préférée moins de 600 ppb, et de manière encore davantage préférée moins de 300 ppb de complexes formés entre la ranitidine et des espèces de fer en poids par rapport au poids sec total de la composition.

3. Composition selon la revendication 1 ou la revendication 2, où ladite composition comprend moins de 320 ppb, de préférence moins de 160 ppb, de manière davantage préférée moins de 80 ppb, et de manière encore davantage préférée moins de 17 ppb de N-nitrosodiméthylamine (NDMA) en poids par rapport au poids sec total de ranitidine ou de ses sels pharmaceutiquement acceptables après une exposition à 70 °C pendant 5 jours.

4. Composition selon l'une quelconque des revendications 1 à 3, dans laquelle le sel pharmaceutiquement acceptable de la ranitidine est le chlorhydrate de ranitidine.

5. Composition selon l'une quelconque des revendications 1 à 4, dans laquelle l'espèce de fer est un fer métallique, un fer (II) et un fer (III) et des mélanges de ceux-ci, de préférence un fer métallique, un oxyde de fer comme un oxyde de fer (II) et un oxyde de fer (III), un chlorure de fer tel que le chlorure de fer (II) et le chlorure de fer (III)) et des mélanges de ceux-ci.

6. Composition selon l'une quelconque des revendications 1 à 5, qui est une composition pharmaceutique.

7. Procédé de préparation de la composition telle que définie dans l'une quelconque des revendications 1 à 6, dans lequel ladite composition n'est pas mise en contact avec des espèces de fer.

8. Procédé de préparation de la composition telle que définie dans l'une quelconque des revendications 1 à 6, dans lequel ladite composition est fabriquée, au moins partiellement, dans un appareil sans acier inoxydable, un appareil en verre, un appareil à ligne en verre, un appareil revêtu de polymères ou de copolymères inactifs comme des copolymères d'éthylène et de chlorotrifluoroéthylène, et du polytétrafluoroéthylène, et/ou un appareil revêtu d'un émail.

9. Procédé de préparation de la composition telle que définie dans l'une quelconque des revendications 1 à 6, qui comprend au moins une étape sélectionnée parmi une étape de complexage d'au moins une partie ou de la totalité des espèces de fer avec un agent chélatant, une étape de suppression d'au moins une partie ou de la totalité des complexes formés entre un agent chélatant et des espèces de fer, et une étape de suppression d'au moins une partie ou de toutes les espèces de fer.

10. Procédé selon la revendication 9, dans lequel l'au moins une étape sélectionnée parmi une étape de complexage d'au moins une partie ou de la totalité des espèces de fer avec un agent chélatant, une étape de suppression d'au moins une partie ou de la totalité des complexes formés entre un agent chélatant et des espèces de fer, et une étape de suppression d'au moins une partie ou de toutes les espèces de fer comprend :
- une étape de distillation de composés de méthylthiométhyle (MTM) tels que le 1-méthylamino-1-méthylthio-2-nitroéthène, et/ou
- une étape de complexation en utilisant du N,N-dialkyl-dithiocarbamate tel que du N,N-diméthyldithiocarbamate, du N,N-diéthyl-dithiocarbamate, et/ou du N,N-dibutyl-dithiocarbamate, de l'acide éthylènediaminetétraacétique (EDTA) et/ou de la cystéamine, facultativement suivie d'une étape de purification, et/ou
- une étape de complexation en utilisant une résine ou silice greffée avec du N,N-dialkyl-dithiocarbamate tel que du N,N-diméthyldithiocarbamate, du N,N-diéthyl-dithiocarbamate, et/ou du N,N-dibutyl-dithiocarbamate, de l'acide éthylènediaminetétraacétique (EDTA) et/ou de la cystéamine, facultativement suivie d'une étape de purification, et/ou
- une étape de complexation en utilisant une résine ou silice greffée avec une chaîne de carbone terminée par un groupe fonctionnel qui est un agent chélatant de fer, facultativement suivie d'une étape de purification, et/ou
- une étape d'adsorption en utilisant de la zéolite, du charbon actif, de la silice ou un composite de charbon actif et de silice, et/ou
- une étape de séparation en utilisant des séparateurs magnétiques.

11. Procédé selon la revendication 10, dans lequel le groupe fonctionnel de l'agent chélatant de fer est choisi parmi un groupe dithiocarbamate, un groupe thiol, un groupe diméthoxytrityle (DMT), un groupe amine, un groupe acide (aminométhyl)phosphonique (AMPA), un groupe cystéine, un groupe propyldiéthanolamine (DEAM), un groupe diamine, un groupe acide dodécane-tétraacétique (DOTA), un groupe imidazole, un groupe acide triaminetétraacétique (TAAcOH), un groupe triaminetétraacétate de sodium (TAACONa), un groupe thiourée, un acide p-toluènesulfonique, un groupe triamine, et des mélanges de ceux-ci.

12. Procédé selon la revendication 10, dans lequel une silice greffée avec une chaîne de carbone terminée par un groupe fonctionnel représente des épurateurs de fer commercialisés par SiliCycle sous le nom commercial SiliaMetS^{®}.

13. Composition selon l'une quelconque des revendications 1 à 6, pour utilisation dans la prévention et/ou le traitement d'ulcères du duodénum, d'ulcères de l'estomac ou gastriques, de brûlures d'estomac, d'une indigestion due à des reflux acides provenant de l'estomac comme des reflux gastro-œsophagiens, de la dyspepsie, de la dermatite et du syndrome de Zollinger-Ellison.
